# EUROPÄISCHE PATENTANMELDUNG

(11) **EP 0 692 227 A1**
(43) Veröffentlichungstag der Anmeldung: **17.01.1996**
(21) Anmeldenummer: 94810408.8
(22) Anmeldetag: 11.07.1994
(51) Int. Cl.: A61F 2/30, A61L 27/00, A61F 2/02

(54) **Flächiges Implantat**

(71) Anmelder: SULZER Medizinaltechnik AG, CH-8404 Winterthur (CH)
(72) Erfinder: Häuselmann, Hans Jörg, Dr. med., CH-3047 Brehmgarten (CH); Paulsson, Mats, Prof. Dr. med., M.E. Müller, CH-3010 Bern (CH); Bittmann, Peter, Dr., CH-8057 Zürich (CH); Thaler, Thomas, Dr., CH-8057 Zürich (CH)
(74) Vertreter: Trieblnig, Adolf

(57) **Zusammenfassung**

Mit der Erfindung sind verschiedene Ausführungen einer zugfesten druckhaltenden Mikromatrix (2) und deren Herstellung, sowie Mechanismen zur Volumenvergrösserung einer druckstabilen Nanomatrix (3) gezeigt, wobei die Nanomatrix (3) in einer formhaltenden Mikromatrix (2) zwischen einer oberen Grenzschicht (4) und einer unteren Grenzschicht (5) eingebettet ist und durch Aufnahme oder Anlagern von Wassermolekülen an neu gebildete Nanomatrix zur Volumenvergrösserung resp. zur Druckerhöhung innerhalb der Mikromatrix (2) führt. Durch Verwendung einer textilen Mikromatrix mit einer Zwischenschicht (6) entsteht ein flächiges unter einem Innendruck stehendes Gebilde, welches ähnliche druckelastische Eigenschaften wie die Knorpelschicht eines Gelenks entwickelt und als Ersatz von defekten Knorpelausschnitten vorgesehen ist.

## Beschreibung

Die Erfindung handelt von einem flächigen Implantat zum Ersatz von defekten natürlichen Gewebeausschnitten mit einer Matrix, die Fasern enthält. Implantate dieser Art werden in der WO 90/12603 gezeigt, wo es um die Herstellung von Knorpel geht. Es werden feste oder flexible Formen einer künstlichen Matrix vorgeschlagen, in denen Zellkulturen in vitro angesetzt werden, welche sich nach einer späteren Verpflanzung in ein Säugetier zu einer knorpelähnlichen Masse entwickeln. Für den Ersatz von Knorpelteilen an Ohr und Nase, wo der Knorpel eine reine Platzhalterfunktion mit geringer mechanischer Belastung einnimmt, haben die aufgezeigten Ausführungen sicher ihre Berechtigung. Hingegen sind Gelenkknorpel oder -knochen grösseren mechanischen Belastungen ausgesetzt, die über eine Platzhalterfunktion hinaus gehen.

Aufgabe der vorliegenden Erfindung ist es ein Implantat zu schaffen, welches weitgehend die druck- und zugelastischen Eigenschaften von natürlichem Gelenkknorpel annimmt wie sie beispielsweise in "Knee Meniscus, Basic and Clinical Foundations", Raven Press Ltd., 1992, Kapitel 2 und 4, oder in "Articular Cartilage and Knee Joint Function, Basic Science and Arthroscopy", Raven Press Ltd., 1990, Kapitel 1 und 2, beschrieben sind.

Diese Aufgabe wird mit dem kennzeichnenden Teil vom unabhängigen Anspruch 1 erfüllt, indem das Implantat aus einer Mikromatrix besteht, welche zunächst aus einer oberen und aus einer unteren Grenzschicht aufgebaut ist. Zwischen den Grenzschichten ist eine Zwischenschicht mit Fasern im Durchmesserbereich von Mikrometern vorgesehen, welche die Grenzschichten auf einem vorgesehenen Abstand halten und innerhalb der Mikromatrix einen Druckaufbau zulassen. Der Druckaufbau entsteht dabei aus einer Nanomatrix mit Elementen in der Grösse von Nanometern, die als vernetzte Strukturen vorliegen können, welche in der Zwischenschicht angesiedelt sind und die über die Grenzschichten wie über eine flüssigkeitsdurchlässige Barrière oder Membran zurückgehalten werden. Ein Beispiel für Elemente einer Nanomatrix wird im Journal of Cell-Science 107, Seiten 17 bis 27 (1994) mit dem Bericht "Phenotypic stability of bovine articular chondrocytes after long-term culture in alginate beads" gegeben.

Die Vorteile der Erfindung liegen darin, dass entsprechend dem Verhalten der Nanomatrix ein knorpelähnliches Polster geschaffen wird, das formbeständig ist und unter Vorspannung steht. Die Eigenschaften dieses Polsters ähneln einem natürlichen Knorpel, indem bei erhöhter Druckbelastung von aussen ein teilweises Zusammensinken unter Abgabe von Wassermolekülen möglich ist, während in den schwach belasteten Perioden ein erneuter Volumen- und - Druckaufbau unter Aufnahme von Wassermolekülen stattfinden kann. Ein weiterer Vorteil liegt darin, dass sich in der Zwischenschicht ein Ueberdruck von bis zu einigen bar, der also weit über den sonst im Gewebe herrschenden physiologischen Drücken liegt, aufbauen kann, und der einem Gleichgewichtszustand zwischen physikalischem Druck und einem Bindungsmechanismus für Wassermoleküle, d.h. einem Quelldruck der teilweise gesättigten Nanomatrixmoleküle entspricht. Im weiteren wird durch den erhöhten Innendruck bei einer knorpelähnlichen Zusammensetzung der Nanomatrix die Bildung von Collagen-Fäserchen und Proteoglycan gefördert. Dies ist vor allem im Bereich der Grenzschichten ein Vorteil und kann soweit gehen, dass die neu gebildeten Collagenstrukturen weitgehend die mechanische Funktion der Mikromatrix in der Grenzschicht und angrenzenden Zwischenschicht übernehmen, wenn die Mikromatrix in der Grenzschicht aus biologisch abbaubarem Material besteht. Als biologisch abbaubar eignen sich zum Beispiel die folgenden Stoffe sowie deren Co- und Mischpolymere: Polymilchsäure, Polyglycolsäure, ε-Caprolacton und Polydioxanon.

Vorteilhafte Weiterbildungen der Erfindung sind in den Ansprüchen 2 bis 27 aufgeführt.

Die Grenzschichten haben in sich eine grössere Dichte als die Mikrofasern im Bereich der Zwischenschicht und sind so aufgebaut, dass sie einer Zugbelastung in der Oberfläche, wie sie sich bei erhöhtem Innen- und Aussendruck ergibt, standhalten. Für die eigentliche Volumenvergrösserung in der Nanomatrix, die zur Druckerhöhung führt, sind verschiedene Mechanismen möglich. In der Nanomatrix können Makromoleküle mit negativen Ladungen eingelagert werden, sodass z.B. aufgrund des Donnan-Effektes Wassermoleküle aus der umgebenden Flüssigkeit in die Nanomatrix einwandern. Eine weitere Möglichkeit der Volumenvergrösserung besteht durch neu synthetisierte extra-zelluläre Matrix der in vitro in die Nanomatrix eingebrachten Zellen und/oder durch Zellwachstum an sich. Bei beiden Methoden kann ein Gel an der Struktur der Nanomatrix beteiligt sein, das einerseits eine Teilfunktion der extra-zellulären Matrix übernimmt und andererseits als Verdünnung beim Aussäen der Zellen wirkt, um eine gewünschte gleichmässige Konzentration beim Einbringen in die Mikromatrix zu erreichen. Daneben garantiert es die phänotypische Stabilität der Zellen, die somit Chondrozyten bleiben. Alginat bildet einen initialen Knorpel-Matrixersatz, in welchem die eingebetteten Zellen ihrem Ursprung entsprechend natürliche Matrixmoleküle bilden.

Eine erhöhte Dichte in der Grenzschicht lässt sich dadurch erzeugen, dass die Mikrofasern der Zwischenschicht in die Grenzschicht umgelegt und dort vernetzt werden. Bei Schnitten in der Grenzschicht und in Ebenen parallel zur Grenzschicht entsteht ein durch Fasern belegter Flächenanteil. Eine Faser, welche in der Zwischenschicht nur einen ihrem Durchmesserquadrat proportionalen Kreisquerschnitt einnimmt, kann in der Grenzschicht einen Querschnitt einnehmen, der zu ihrem Durchmesser und zu der in der Grenzschicht ausgelegten Länge proportional ist. Dabei ist es von Vorteil, wenn die Mikrofasern der Zwischenschicht in einem Bogen in eine Grenzschicht übergehen, weil dann die in der Nanomatrix eingebetteten Fasern bei einer Druckbelastung von aussen nicht nur gestaucht, sondern auch auf Biegung und Zug belastet werden, wenn die Grenzschicht nach innen nachgibt.

Die Grenzschicht kann auch aus einer flüssigkeitsdurchlässigen Membran bestehen, die mit den Mikrofasern der Zwischenschicht verbunden ist, wobei zum Beispiel die als Flor ausgeführten Mikrofasern zur Membran hin thermisch angeschmolzen und mit dieser verschweisst werden.

Weitere Möglichkeiten für eine Grenzschicht ergeben sich mit einem Gewebe oder Gewirk, das mit den Mikrofasern der Zwischenschicht verschweisst ist. Die Schweisszonen können beispielsweise dadurch erzeugt werden, dass man Zwischenschicht und Grenzschicht im Verbund über eine Nadeltrommel mit beheizten Nadeln zieht, um mit den eintauchenden Nadelspitzen Schweisspunkte zu erzeugen. Eine Verbindung der beiden Schichten kann aber auch durch Anlösen mit einem Lösungsmittel, durch Kleben mit einem körperverträglichen Kleber wie einem FIBRIN KLEBER (TISSUCOL ® oder wie Gewebetransglutaminase, einem Vernetzungsenzym) oder durch Vernähen mit einem biokompatiblen Faden erfolgen. Wenn die Verbindung wie beim Nähen jedoch grossflächig über beide Grenzschichten mit Mikrofäden erfolgt, kann die Zwischenschicht auch ein Vlies als Abstandshalter aufnehmen.

Besonders vorteilhaft sind Ausführungsformen einer Mikromatrix, die sich in einem Arbeitsgang herstellen lassen. So ist eine in zwei Ebenen auf einem Bandwebstuhl für Samtbänder gewobene Mikromatrix mit zwei dichteren Grenzschichten herstellbar, wenn die beim Samt übliche Schneidoperation weggelassen wird. Die Mikrofasern der Zwischenschicht werden aus den Polfäden gebildet, während in der oberen und unteren Webebene die Grenzschichten der Mikromatrix entstehen. Dabei können sich die Kettfäden einer Ebene in ihrem Titer, in der Anzahl der Filamente und im Werkstoff unterscheiden. So können bestimmte Kettfäden auch metallisch z.B. aus Titan sein, um ein Einwachsen bei Knochenmaterial zu fördern.

Eine weitere spezielle Herstellung der Mikromatrix besteht im Wirken in zwei Ebenen wie zum Beispiel auf einer doppelbarrigen Raschelmaschine.

Die Fasern der Mikromatrix müssen körperverträglich sein und können zum Beispiel aus Polyethylen-Terephtalat, Polyetherketon, Polypropylen, Teflon, Kohlenstoff oder Polyethylen bestehen. Wenn die gleichen Materialien als Fäden mit vielen Fibrillen in den Grenzschichten verarbeitet sind wie beim Weben in zwei Ebenen, entstehen recht feine Siebstrukturen, um die Nanomatrix aufzunehmen. Diese kann nun Makromoleküle in der Art der Proteoglycan-Aggregate mit Molekulargewichten von 50 bis 100 Millionen Dalton aufweisen, um das Volumen und die Wasseraufnahme in der Nanomatrix zu steuern. Sie kann aber auch mit Zellstrukturen vom Typ der Chondrozyten versehen sein, um durch neu gebildete Nanomatrix Volumen- und Druckänderungen zu erzeugen. Im folgenden wird die Erfindung anhand von Ausführungsbeispielen beschrieben. Es zeigen:
- Fig. 1: schematisch den Aufbau einer Mikromatrix mit Zwischenschicht sowie unterer und oberer Grenzschicht;
- Fig. 2: schematisch die Bindung bei der Herstellung einer Mikromatrix auf einem Bandwebstuhl für Samt, bei dem die Schneidoperation weggelassen wird;
- Fig. 3: schematisch eine andere Art der Bindung bei der Herstellung eines Zweiwandgewebes mit Kettfadensystem und mit Doppelgreifern;
- Fig. 4: schematisch die Anordnung von Zungennadeln und Legebarren bei der Herstellung von Maschenschneidplüsch auf einer doppelbarrigen Raschelmaschine;
- Fig. 5: schematisch den Nähwirkzyklus bei der Herstellung einer Mikromatrix aus einem Vlies in Nähwirktechnik;
- Fig. 6a: schematisch das dreidimensionale Netzwerk einer Nanomatrix, z.B. Alginat, bei dem die Kettenassoziation durch Komplexbildung mit Calcium-Ionen erfolgt;
- Fig. 6b: schematisch die Chelatbindungen von Fig. 6a in einer in der Chemie üblichen Darstellung;
- Fig. 7: schematisch einen Ausschnitt aus einer Zwischenschicht mit Mikrofaser und Nanomatrix, welche Zellen mit extrazellulärer Matrix und Collagenfäserchen aufweisen;
- Fig. 8: schematisch einen vergrösserten Ausschnitt von Fig. 7, bei dem die interagierenden Collagen- und Proteoglycannetzwerke gezeigt sind;
- Fig. 9a, b: schematisch die Herstellung einer Membran an einem Vlies oder Flor;
- Fig. 10: schematisch einen Ausschnitt aus einer oberen Grenzschicht mit einer Nanomatrix, welche vernetzte Zellen mit extrazellulärer Matrix aufweist.

In den Figuren sind verschiedene Ausführungen einer zugfesten druckhaltenden Mikromatrix 2 und deren Herstellung, sowie Mechanismen zur Volumenvergrösserung einer druckstabilen Nanomatrix 3 gezeigt, wobei die Nanomatrix 3 in einer formhaltenden Mikromatrix 2 zwischen einer oberen Grenzschicht 4 und einer unteren Grenzschicht 5 eingebettet ist und durch Aufnahme oder Anlagern von Wassermolekülen an neu gebildete Nanomatrix zur Volumenvergrösserung resp. zur Druckerhöhung innerhalb der Mikromatrix 2 führt. Durch Verwendung einer textilen Mikromatrix mit einer Zwischenschicht 6 entsteht ein flächiges unter einem Innendruck stehendes Gebilde, welches ähnliche druckelastische Eigenschaften wie die Knorpelschicht eines Gelenks entwickelt und als Ersatz von defekten Knorpelausschnitten vorgesehen ist.

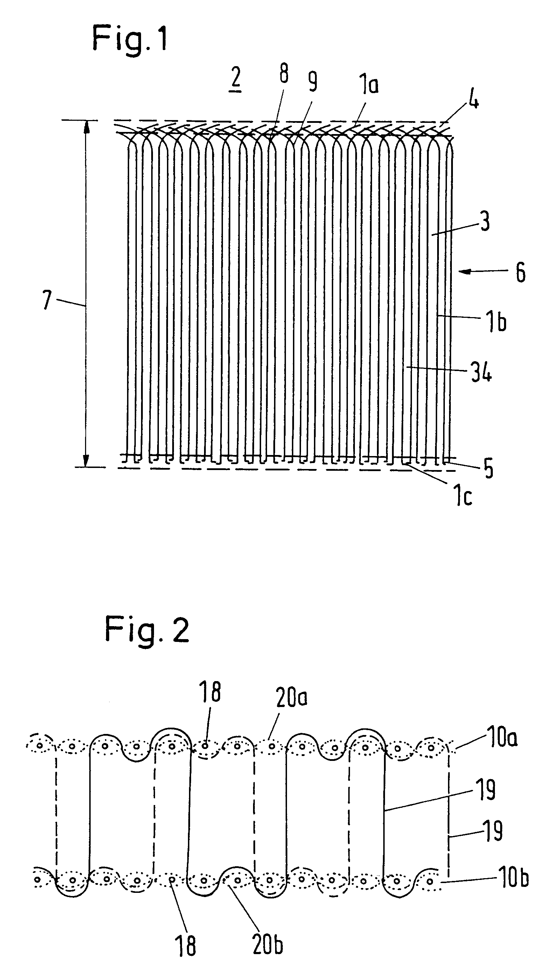

In Figur 1 ist eine Mikromatrix 2 mit einer oberen Grenzschicht 4, in der Fasern 1a liegen, mit einer Zwischenschicht 6, welche von Fasern 1b überbrückt wird, und mit einer unteren Grenzschicht 5, in der Fasern 1c liegen, gezeigt. Obere und untere Grenzschichten 4, 5 sind durch die Fasern 1b der Zwischenschicht 6 auf einem konstanten Abstand 7 von beispielsweise 2 mm gehalten. In der Zwischenschicht 6 und bis in die Grenzschichten 4, 5 hinein ist eine neu gebildete Nanomatrix eingebettet, die durch Aufnahme oder Anlagern von Wassermolekülen eine Volumenvergrösserung bewirkt und zur Erhöhung vom Innendruck 34 in der Zwischenschicht 6 führt. Die Fasern 1b gehen in einem Bogen 9 in Fasern 1a der oberen Grenzschicht 4 über und tragen so zur Verdichtung der oberen Grenzschicht bei. Diese muss im Verbund mit der Nanomatrix soviel Zugfestigkeit in ihrer Oberfläche aufweisen, dass sie dem Innendruck 34 und auch dem sich ergebenden Druck bei stossartiger Belastung von aussen standhalten kann. Die Herstellung einer solchen Mikromatrix kann auf unterschiedliche Weise erfolgen. Wenn Mikrofasern 1a, 1b, 1c an der Bildung beteiligt sind, dann weisen sie mit Vorteil mehrere Fibrillen und eine Verdrillung von wenigen Umdrehungen pro Meter auf, um an den Umlenkungen in der Grenzschicht einen eher flachen linsenförmigen Querschnitt zu erhalten.

Ein Bandwebstuhl für Samt bringt die geforderten feinen Strukturen zustande. Figur 2 zeigt Bandweben in einer oberen Ebene 10a und einer unteren Ebene 10b, die jeweils den Grenzschichten 1a, 1c entsprechen, wobei die Kettfäden 20a, 20b in diesen Ebenen verlaufen und durch Schussfäden 18 abgebunden werden. Polfäden 19, welche die obere und untere Ebene 10a, 10b verbinden, bilden die Fasern 1b in der Zwischenschicht. Bandwebstühle für Samt, die ein solches Gewebe herstellen können, werden beispielsweise bei der Firma Jacob Müller AG, CH-5262 Frick (Schweiz) gefertigt. Durch Verzicht auf die beim Samt notwendige Schneidoperation entsteht ein doppelwandiges Gewebe, welches durch die Polfäden zusammengehalten wird. Dabei sind auch Kettfäden 20 mit mehreren Fibrillen und in verschiedenen Materialien möglich. So können beispielsweise auch metallische Kettfäden 20b in der unteren Ebene 10b verarbeitet werden, die, wenn sie in Titan ausgeführt sind, eine für das Anwachsen an Knochen günstige Textur aufweisen.

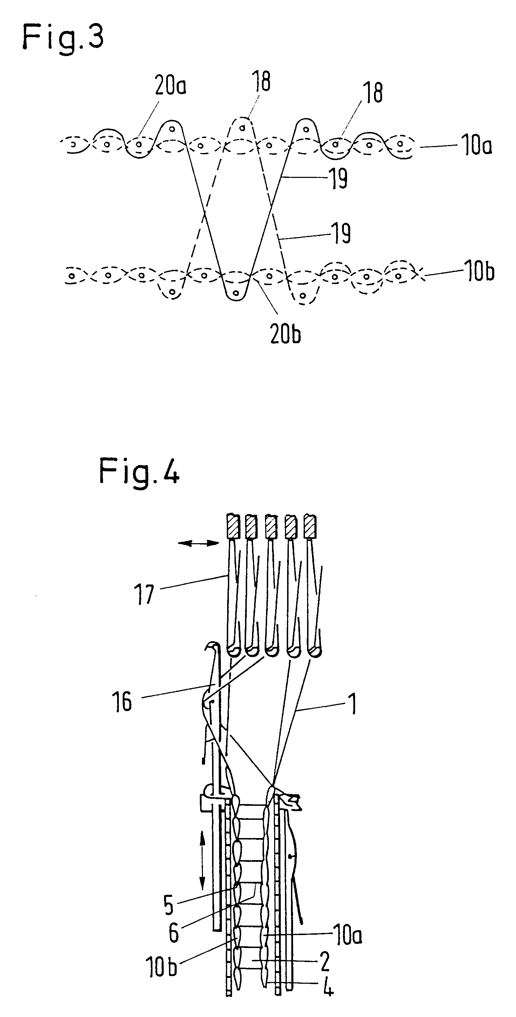

Aehnlich ist die Situation bei einer Mikromatrix entsprechend Figur 3, die auf einer miniaturisierten Ausführung einer Webmaschine mit einem Kettfadensystem und Doppelgreifern für Zweiwandgewebe hergestellt wird, wobei wie beim Bandwebstuhl nur geringe Breiten bis zu 50 mm notwendig sind, um Auswechselstücke für natürlichen Knorpel zu erhalten. Es wird ebenfalls mit oberen Kettfäden 20a in einer oberen Ebene 10a und mit unteren Kettfäden 20b in einer unteren Ebene 10b gearbeitet, wobei zusätzliche Polfäden 19 über Schüsse 18 mit in die Kettfäden 20a, 20b eingebunden sind.

In Figur 4 ist die Herstellung einer gewirkten Mikromatrix 2 auf einer doppelbarrigen Raschelmaschine gezeigt, welche sonst zur Herstellung von Maschenschneidplüsch verwendet wird. In einer oberen und unteren Ebene 10a, 10b werden jeweils über Zungennadeln 16 und Legebarren 17 die Fasern 1 zu einer oberen und unteren Grenzschicht 4, 5 und zu einer Zwischenschicht 6 verarbeitet.

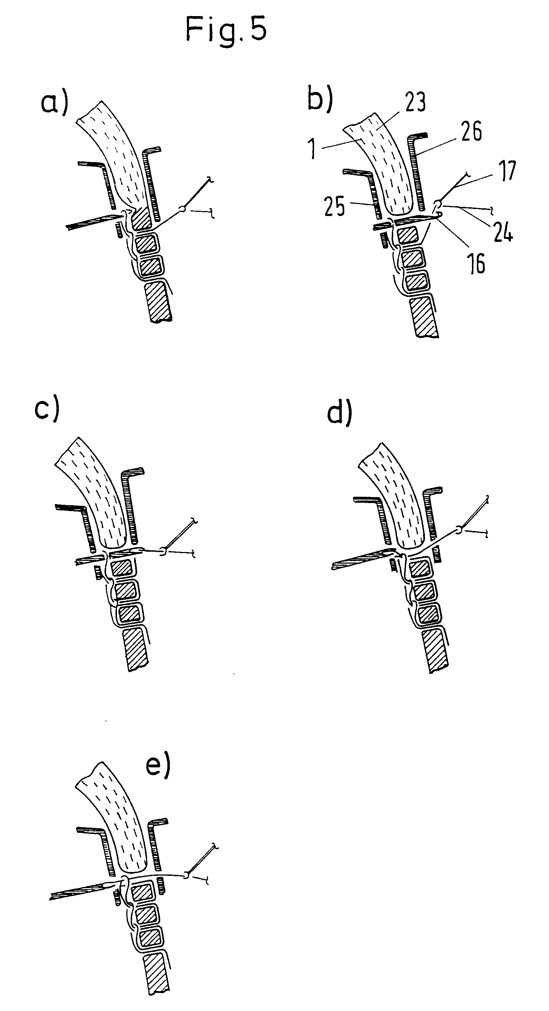

Bei einer weiteren Herstellmöglichkeit einer Mikromatrix 2 wird von einem Vlies 23 ausgegangen, das über Nähwirken zu einer Ratte verarbeitet wird. In Figur 5 ist der prinzipielle Nähwirkzyklus beschrieben. Ein Vlies 23 wird über trichterförmige Begrenzungen 25, 26 geführt und mit Fäden 24 über Nadeln 16 und Legebarren 17 zu einer Matte vernäht. Die Arbeitsgänge sind a) Durchstechen, b) Legen, c) Verschliessen der Nadelköpfe, d) Abschlagen, e) Abzug der Matte. Die relativ weiten Maschen der Nähfäden sind dabei als Unterstützung vom Vlies in Querrichtung gedacht, wobei weitere Abstützungen zwischen Berührungspunkten 8 von Fasern 1 im Vlies vorgesehen sind, die durch Anlösen mit einem Lösungsmittel oder durch Verkleben mit einem Kleber erzeugt werden und bis in die Zwischenschicht hinein entstehen.

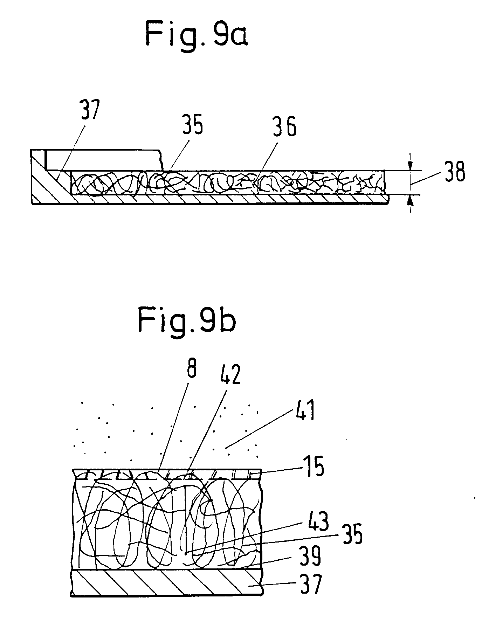

Eine spezielle obere Grenzschicht in Form einer Membran 15 mit Poren 42 in der Grösse von 5 bis 10 µm wird mit Polyurethan gemäss Figuren 9a, 9b erzeugt. Ein Vlies 35, ein Gewirk oder ein Gewebe mit einer Höhe 38 von beispielsweise 3 mm wird in einer Schale 37 ausgelegt und diese bis zu den obersten Fasern mit Dimethylformamid und 5 % bis 10 % darin gelösten Polyurethan aufgefüllt. Ueber der Lösung wird eine feuchte Atmosphäre aufrechterhalten, die dazu führt, dass sich das stark hygroskopische Dimethylformamid in der Grenzschicht mit Wassermolekülen versorgt und dort gleichzeitig die Löslichkeit von Polyurethan herabsetzt, um so eine Schwammfläche aus Polyurethan in Form einer Membran mit einer Mikrostruktur zu erzeugen. Auch die tiefer am Boden 39 der Schale 37 befindlichen Polyurethanteilchen 43 sind bei Temperaturen von ca. 50°C so beweglich, dass sie in die obere Grenzschicht einwandern. Es entsteht so eine Grenzschicht von bis zu 0,3 mm Dicke, die Poren 42 aufweist und die Fasern von der Zwischenschicht einbindet. Durch anschliessende Lyophylisation kann die Porenstruktur in der oberen Grenzschicht erhalten bleiben. Eine solche Membran 15 unterstützt die Nanomatrix bei der Bildung von einem Innendruck. Sie hat für eine Nanomatrix mit extrazellulärer Matrix, die Proteoglycan-Aggregate und Collagenfäserchen enthält, den Vorteil, dass sie, wenn sie aus körperverträglichen, resorbierbarem Material hergestellt wird, anfänglich unterstützt, sich aber entsprechend der wachsenden Tragfähigkeit der sich in der oberen Grenzschicht bildenden Collagenstruktur zurückbildet, d.h. von der Körperflüssigkeit im implantierten Zustand langsam aufgelöst wird.

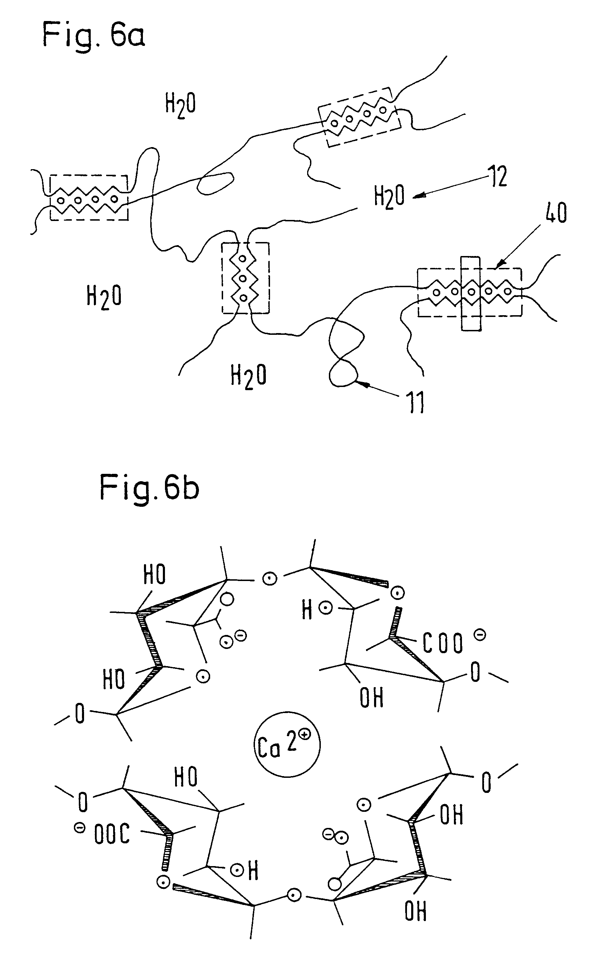

An dem Aufbau einer Nanomatrix sind Hydrogele beteiligt, die als hydrophile Makromoleküle linear oder verzweigt vorkommen. Die dreidimensionalen Netzwerke entstehen dadurch, dass regelmässig geordnete Zonen von Kettenmolekülen 11 zu sogenannten Haftzonen 40 zusammentreten. Ein so gebildetes Hydrogel hat den Vorteil, dass es für kleine Moleküle wie zum Beispiel Metabolite oder Wasser durchlässig ist. Die Bindung von Wassermolekülen und damit das Volumen des Hydrogels hängt von den fixen Ladungen des Hydrogels ab. Ein Beispiel für Bausteine mit fixen Ladungen bilden die Alginate, die als Polysaccharid aus Manuronsäure und Guluronsäure in wechselnden Blocksequenzen aufgebaut sind. Bei gewissen Polysacchariden (z.B. Alginate) kommen die Haftzonen 40 so zustande, dass Manuronsäure-Einheiten, die wegen der axial-axial glykosidischen Bindung in Zickzack-Ketten vorliegen, einander gegenüberliegen. Diese Anordnung wird dann durch Calcium-Ionen, welche Bindungen mit den Carboxyl- und den Hydroxylgruppen eingehen, fixiert. Dies ist in der Figur 6b, in der üblichen chemischen Schreibweise, schematisch dargestellt. In Figur 6a ist eine solche Vernetzung schematisch mit den frei beweglichen Wassermolekülen dargestellt, welche als mögliche Dipole beim Auftreten von fixen Ladungen zu Verfügung stehen. In Figur 6b ist der gleiche Vorgang als Struktur in chemischer Schreibweise dargestellt. Durch gesteuerte Vernetzung und durch Aufnahme oder Anlagern von Wassermolekülen lässt sich das Volumen einer Nanomatrix innerhalb der Mikromatrix vergrössern, um einen Innendruck zu erzeugen.

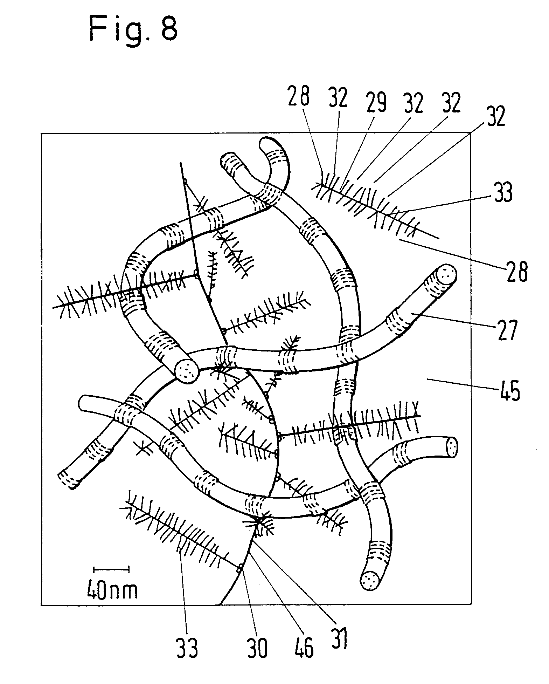

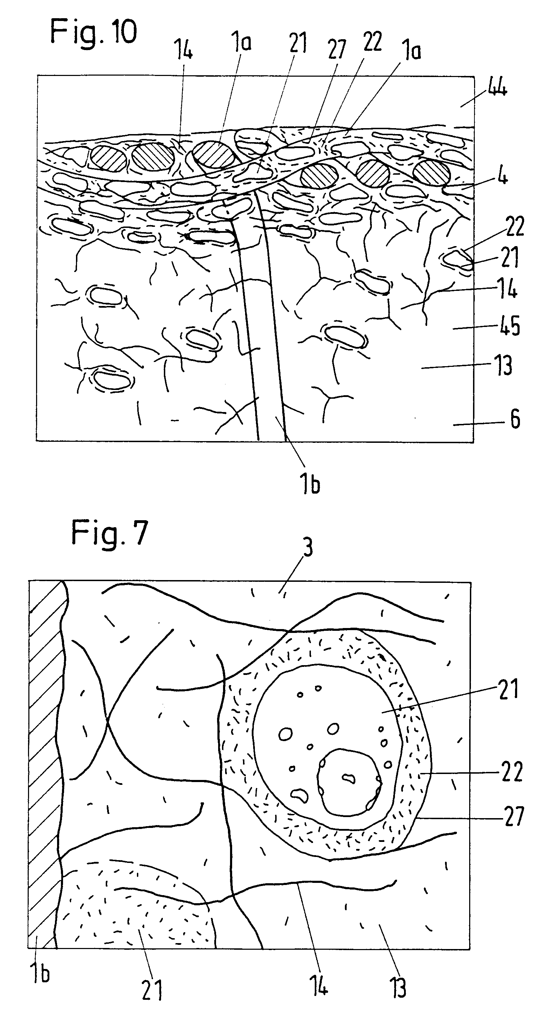

Eine Mikromatrix 2 nach Figur 1, in die eine Nanomatrix 3 eingelagert ist, hat den Vorteil, dass die Fasern 1b in der Zwischenschicht einer Flüssigkeitsverlagerung Widerstand entgegensetzen, sodass eine schlagartige Druckbelastung von aussen hauptsächlich längs den Fasern 1b in der Zwischenschicht weitergegeben wird. Trotzdem muss am Rand des Implantats eine ähnlich dichte Verbindung, wie sie in einer Grenzschicht 4, 5 selbst besteht, angebracht werden. Durch Vernähen der Ränder, durch Verkleben der Ränder oder durch Anbringen von Stützringen entstehen ravioliähnliche Polster, die mit einem Gel 13, z.B. aus Alginat oder aber mit einem Gel 13 und Zellen 22, welche extrazelluläre Matrix bilden, bestückbar sind. Figur 7 zeigt einen Ausschnitt aus der Zwischenschicht 6 eines solchen Polsters. Eine angeschnittene Faser 1b liegt innerhalb des Gels 13, in welches Collagenfasern 14 und Zellen 21 mit extrazellulär gewachsener Matrix 22, die ihrerseits Collagenfäserchen 27 enthält, eingelagert sind. Allein das Wachsen der extrazellulären Matrix 22 erzeugt in wenigen Wochen in vitro eine respektable Volumenvergrösserung innerhalb eines Polsters. Die Zellen 21 sind Chondrozyten, die möglichst gleichmässig in der vorläufig nur mit Alginat bestückten Nanomatrix 3 verteilt sind. Mit zunehmendem Wachstum der Nanomatrix werden Proteoglycan-Aggregate 46 und Collagenfasern 14 eingelagert, welche eine zusätzliche Stütz- und Zugfunktion haben, die besonders im Bereich der Grenzschicht 4, siehe Figur 10, zum Tragen kommt, weil dort eine Verdichtung von Chondrozyten und von Collagenfasern in extrazellulärer Matrix stattfindet, indem dort die kürzesten Wege zur aussenliegenden Nährflüssigkeit 44 und im implantierten Zustand zur Synovialflüssigkeit bestehen.

Figur 8 zeigt eine Vergrösserung der extrazellulären Matrix 22, die von Proteoglycan-Aggregaten 46 und Collagenfäserchen 27 durchsetzt ist. Wassermoleküle 12 können sich initial frei bewegen. Ein kleiner Teil des Wassers ist in den Collagenfasern 27 gebunden, die z.B. in Form einer "triple helix" ausgebildet sind. Als weitere Komponente der extra-zellulären Matrix sind Proteoglycan-Monomere 33 gezeigt, bestehend aus einem Proteinfaden (Protein Core) 29 und daran gekoppelte lineare Polysaccharide 28, die negative Gruppen 32 (SO₃ ^{⊖}, COO ^{⊖}) tragen. Die Proteoglycan-Monomere 33 wiederum sind über Link-Proteine 30 an Hyaluronsäure-Fäden 31 gebunden, die gesamthaft das Proteoglycan-Aggregat 46 als Netzwerk ausbilden. Sie sind es vor allem, die Wasser binden.

Basierend auf dem Prinzip der Elektroneutralität werden entsprechend dem Vorliegen von gebundenen negativen Ladungen positive Ionen z.B. Na ^{⊕} in die extra-zelluläre Matrix aufgenommen. Dadurch liegen innerhalb des Gewebes mehr osmotisch aktive Teilchen vor als in der umgebenden Körperflüssigkeit. Diese Ungleichheit der Ionenkonzentration führt zu einem osmotischen Druck zwischen der interstitiellen Flüssigkeit 45 und der umgebenden Körperflüssigkeit, der bei begrenztem Volumen zur Durckerhöhung führt. Das Molekulargewicht der Proteoglycan-Aggregate bewegt sich in der Grösse von 50 bis 100 Millionen Dalton (Da). Das Proteoglycan-Monomer 33, welches aus Protein-Core 29 und Polysacchariden 28 besteht, weist 1 Million Dalton auf, während kettenförmige Hyaluronsäure etwa 2 bis 6 Millionen Dalton entspricht. Bis zu 100 Millionen Dalton können entstehen, wenn sich lange Ketten 31 von Hyaluronsäure mit daran gebundenen Proteoglycan-Aggregaten bilden.

## Patentansprüche

1. Flächiges Implantat zum Ersatz von defekten natürlichen Gewebeausschnitten mit einer Matrix, die Fasern (1) enthält, dadurch gekennzeichnet, dass es aus einer Nanomatrix (3) mit Elementen in der Grösse von Nanometern und aus einer Mikromatrix (2) besteht, welche aus einer oberen und einer unteren Grenzschicht (4, 5) aufgebaut ist, die über eine Zwischenschicht (6), welche die Nanomatrix (3) aufnimmt, derart mit Fasern (1) im Durchmesserbereich von Mikrometern verbunden sind, dass bei wachsendem Innendruck zwischen den Grenzschichten (4, 5) diese in einem vorgegebenen Abstand (7) zueinander haltbar sind.

2. Implantat nach Anspruch 1, dadurch gekennzeichnet, dass bei der Mikromatrix (2) die Grenzschichten (4, 5) im Mittel eine höhere Dichte als die Zwischenschicht (6) aufweisen, um bei wachsendem Volumen der Nanomatrix (3) einen Innendruck zu erzeugen, wobei für den Dichtevergleich nur die Fasern (1b) der Mikromatrix (2) in der Zwischenschicht (6) zu berücksichtigen sind.

3. Implantat nach Anspruch 1 oder 2, dadurch gekennzeichnet, dass in der Nanomatrix (3) Kettenmoleküle (11) eingelagert sind, die das Volumen der Nanomatrix (3) durch Aufnahme von Wassermolekülen (12) vergrössern.

4. Implantat nach Anspruch 1 oder 2, dadurch gekennzeichnet, dass in der Nanomatrix (3) in vitro eingelagerte Zellen (21) vorhanden sind, die das Volumen der Nanomatrix (3) über neu synthetisierte extrazelluläre Matrix (22) vergrössern.

5. Implantat nach Anspruch 3 oder 4, dadurch gekennzeichnet, dass an der Struktur der Nanomatrix (3) ein Gel (13) beteiligt ist, das die eingelagerten Zellen phänotypisch stabil hält.

6. Implantat nach einem der Ansprüche 1 bis 5, dadurch gekennzeichnet, dass die Fasern (1b) der Zwischenschicht (6) untereinander Verbindungen an Berührungspunkten (8) aufweisen.

7. Implantat nach einem der Ansprüche 1 bis 5, dadurch gekennzeichnet, dass mindestens eine Grenzschicht (4, 5) umgelegte Fasern (1b) der Zwischenschicht (6) aufweist, welche durch ihren Verlauf in der Grenzschicht (4, 5) und durch ihre Verbindung in dieser Grenzschicht eine Schicht grösserer Dichte im Vergleich zur Faserdichte der Zwischenschicht (6) bilden.

8. Implantat nach Anspruch 7, dadurch gekennzeichnet, dass die Fasern (1b) der Zwischenschicht (6) in einem Bogen (9) aus der Zwischenschicht (6) in eine Grenzschicht (4, 5) einlaufen.

9. Implantat nach einem der Ansprüche 1 bis 6, dadurch gekennzeichnet, dass mindestens eine der beiden Grenzschichten (4, 5) aus einer flüssigkeitsdurchlässigen Membran (15) besteht.

10. Implantat nach Anspruch 9, dadurch gekennzeichnet, dass die Membran (15) aus Polyurethan gebildet ist.

11. Implantat nach einem der Ansprüche 1 bis 8, dadurch gekennzeichnet, dass mindestens eine der beiden Grenzschichten (4, 5) aus einem Gewebe oder Gewirk gebildet ist.

12. Implantat nach Anspruch 11, dadurch gekennzeichnet, dass das Gewebe oder Gewirk aus körperverträglichem Fasermaterial wie Polyethylen-Terephtalat, Polyetherketon, Polypropylen, Teflon, Kohlenstoff oder Polyethylen gebildet ist.

13. Implantat nach einem der Ansprüche 1 bis 8 oder 11 bis 12, dadurch gekennzeichnet, dass es durch Weben in zwei Ebenen (10a, 10b), insbesondere durch Weben auf einem Bandwebstuhl für Samt unter Weglassen der Schneidoperation, herstellbar ist.

14. Implantat nach einem der Ansprüche 1 bis 8 oder 11 bis 12, dadurch gekennzeichnet, dass es durch Wirken in zwei Ebenen (10a, 10b) wie zum Beispiel auf einer doppelbarrigen Raschelmaschine herstellbar ist.

15. Implantat nach Anspruch 13 oder 14, dadurch gekennzeichnet, dass sich Kettfäden (20a) einer Ebene (10a) von Kettfäden (20b) in der anderen Ebene (10b) durch ihren Titer oder die Zahl der Filamente in ihrem Querschnitt unterscheiden.

16. Implantat nach Anspruch 13, dadurch gekennzeichnet, dass sich die Kettfäden (20a) einer Ebene (10a) von den Kettfäden (20b) in der anderen Ebene (10b) durch ihren Werkstoff unterscheiden.

17. Implantat nach Anspruch 15, dadurch gekennzeichnet, dass in einer Ebene Kettfäden aus Metall vorzugsweise aus Titan sind.

18. Implantat nach einem der Ansprüche 1 bis 12, dadurch gekennzeichnet, dass die Zwischenschicht (6) aus einem Gewebe, Gewirk oder Vlies (23) besteht.

19. Implantat nach einem der Ansprüche 1 bis 10, dadurch gekennzeichnet, dass die Verbindung zwischen Zwischenschicht (6) und einer Grenzschicht (4, 5) durch Verschweissen erfolgt.

20. Implantat nach einem der Ansprüche 1 bis 10, dadurch gekennzeichnet, dass die Verbindung zwischen Zwischenschicht (6) und einer Grenzschicht (4, 5) durch Anlösen mit einem Lösungsmittel oder durch Verkleben mit einem Kleber erfolgt.

21. Implantat nach einem der Ansprüche 1 bis 12, dadurch gekennzeichnet, dass die Verbindung zwischen Zwischenschicht (6) und einer Grenzschicht (4, 5) durch Vernähen erfolgt.

22. Implantat nach einem der Ansprüche 1 bis 21, dadurch gekennzeichnet, dass die Nanomatrix (3) Collagenfasern (14) enthält.

23. Implantat nach Anspruch 22, dadurch gekennzeichnet, dass der Anteil an Collagenfasern (14) in der Nanomatrix bis zu 15 % der Trockensubstanz ausmacht.

24. Implantat nach Anspruch 3, dadurch gekennzeichnet, dass die in der Nanomatrix (3) eingelagerten Proteoglycan-Aggregate 46 Molekulargewichte von 50 bis 100 Millionen Dalton aufweisen.

25. Implantat nach Anspruch 3 oder 24, dadurch gekennzeichnet, dass die Kettenmoleküle (11) aus Verbindungen der Art der Polysaccharide bestehen.

26. Implantat nach Anspruch 4, dadurch gekennzeichnet, dass die eingelagerten Zellstrukturen (21) von Typ der Chondrozyten sind.

27. Implantat nach einem der Ansprüche 1 bis 26, dadurch gekennzeichnet, dass die Zwischenschicht (6) und/oder eine Grenzschicht (4, 5) der Mikromatrix (2) aus biologisch abbaubarem Material wie zum Beispiel aus Polymilchsäure, Polyglycolsäure, ε-Caprolacton, Polydioxanon sowie aus Co- und Mischpolymeren dieser Verbindungen bestehen.

28. Verwendung des Implantats nach einem der Ansprüche 1 bis 27 für die Herstellung von künstlichem Knorpel.
